# EUROPEAN PATENT SPECIFICATION

(11) **EP 3 894 523 B1**
(45) Date of publication and mention of the grant of the patent: **07.06.2023**
(21) Application number: 19809131.6
(22) Date of filing: 15.11.2019
(51) Int. Cl.: C10L 1/08, C10L 1/185, C10G 3/00, C10G 45/58, C10G 65/04, C07C 41/05, C10L 10/08

(54) **DIESEL FUEL COMPOSITION**
DIESELKRAFTSTOFFZUSAMMENSETZUNG
COMPOSITION DE CARBURANT DIESEL

(30) Priority: 14.12.2018 FI 20186078
(43) Date of publication of application: 20.10.2021
(73) Proprietor: Neste Oyj, 02150 Espoo (FI)
(72) Inventor: KIISKI, Ulla, 06101 PORVOO (FI); HEISKA, Arto, 06101 Porvoo (FI); KOUVA, Merja, 06101 Porvoo (FI); AALTO, Mikko, 06101 Porvoo (FI); KURONEN, Markku, 06101 Porvoo (FI)
(74) Representative: Espatent Oy
(86) International application number: PCT/FI2019/050814
(87) International publication number: WO 2020/120832

(56) References cited:
- WO-A1-2013/038029
- WO-A2-2009/066925
- WO-A2-2010/077476
- US-A- 5 308 365
- US-A- 6 015 440
- A. JAECKER-VOIROL ET AL: "Glycerin for New Biodiesel Formulation", OIL & GAS SCIENCE AND TECHNOLOGY - REVUE DE L'INSTITUT FRAN?AIS DU P?TROLE, vol. 63, no. 4, 1 July 2008 (2008-07-01), pages 395-404, XP055056176, ISSN: 1294-4475, DOI: 10.2516/ogst:2008033

## Description

### TECHNICAL FIELD

The present invention generally relates to a diesel fuel composition. The invention relates particularly, though not exclusively, to a diesel fuel composition comprising a renewable diesel component and di-glycerol tert-butyl ether and having beneficial lubricity properties. Further, the invention relates, though not exclusively, to use of di-glycerol tert-butyl ether as a lubricity improver.

### BACKGROUND ART

This section illustrates useful background information without admission of any technique described herein representative of the state of the art.

Environmental considerations have in recent years impacted the fuel industry, which has traditionally relied on fuels of fossil origin. New fuels and fuel blending components of renewable origin have been introduced as more sustainable alternatives to fossil fuels.

One such renewable component is biodiesel (fatty acid methyl esters, FAME), the production of which has increased lately. A by-product of biodiesel production is glycerol and with the increased production of biodiesel, also the availability of renewable glycerol has increased. As a consequence, there is a need to find value adding applications for the glycerol from the biodiesel production process.

Another renewable fuel component is renewable diesel, sometimes also referred to as hydrotreated vegetable oil. An advantage of renewable diesel over biodiesel is that current diesel fuel standards (EN 590:2017) do not restrict the amount of renewable diesel in diesel fuels enabling a high amount of renewable components in diesel fuel, whereas the amount of biodiesel in diesel fuels is restricted to a maximum of 7 vol-%. However, neat non-additized renewable diesel - like fossil ultra low sulphur diesel - does not have desired lubricity properties. Non-satisfactory lubricity properties subject engines to excessive wear and possible damages.

US 5308365 relates to a diesel fuel which has reduced particulate matter emission characteristics and which contains an effective amount of a dialkyl ether and/or trialkyl ether derivative of glycerol.

WO2010/077476 relates to a process for controlling the cloud point of transportation fuels including diesel fuel and aviation fuel from renewable feedstocks.

WO2009/066925 relates to a method of manufacturing glycerol ether, and a fuel composition for an automobile including the glycerol ether.

### SUMMARY

The appended claims define the scope of protection. Any examples and technical descriptions of products, methods and/or uses in the description and/or drawings not covered by the claims are presented not as embodiments of the invention but as examples useful for understanding the invention.

According to a first aspect of the invention there is provided a diesel fuel composition comprising:
a. a renewable paraffinic diesel component at most 95 vol-% of the total diesel fuel composition volume, the renewable paraffinic diesel component comprising paraffins in the range of carbon number C15-C18 at least 70 wt-% of the total weight of paraffins in the renewable paraffinic diesel component; and
b. di-glycerol tert-butyl ether (di-GTBE) at least 5 vol-% of the total diesel fuel composition volume.

It has surprisingly been found that diesel fuel compositions comprising a renewable paraffinic diesel component and at least 5 vol-% di-GTBE have beneficial lubricity properties.

In certain embodiments, the diesel fuel composition comprises di-GTBE 5-50 vol-% or 5-45 vol-%, preferably 5-40 vol-% or 5-35 vol-%, further preferably 5-30 vol-%, yet further preferably 5-25 vol-%, more preferably 5-20 vol-%, and even more preferably 5-15 vol-% of the total diesel fuel composition volume. In certain embodiments, the diesel fuel composition comprises di-GTBE 5-22 vol-%, preferably 5-18 vol-% or 5-16 vol-%, further preferably 5-13 vol-%, more preferably 7-11 vol-%, even more preferably 8-10 vol-% of the total diesel fuel composition volume. Diesel fuel compositions comprising such vol-% di-GTBE have beneficial lubricity properties and provide good compatibility with diesel fuel engines. The High Frequency Reciprocating Rig (HFRR) -lubricity according to EN ISO12156-1:2016 of such fuels is 460 µm or less.

In certain embodiments, the diesel fuel composition comprises at least 10 vol-% di-GTBE and at most 90 vol-% renewable paraffinic diesel component of the total fuel composition volume. In certain embodiments, the diesel fuel composition comprises di-GTBE 10-50 vol-% or 10-45 vol-%, preferably 10-40 vol-% or 10-35 vol-%, further preferably 10-30 vol-%, yet further preferably 10-25 vol-%, more preferably 10-20 vol-%, and even more preferably 10-15 vol-% of the total diesel fuel composition volume. In certain embodiments, the diesel fuel composition comprises di-GTBE 10-22 vol-% or 10-19 vol-%, preferably 10-17 vol-% or 10-16 vol-%, more preferably 10-13 vol-% and even more preferably 10-12 vol-% of the total diesel fuel composition volume. Diesel fuel compositions comprising such vol-% di-GTBE have particularly beneficial lubricity properties and provide good compatibility with diesel fuel engines. The (HFRR) -lubricity according to EN ISO12156-1:2016 of such diesel fuel compositions is 400 µm or less.

In certain embodiments, the diesel fuel composition comprises renewable paraffinic diesel component 50-95 vol-% or 50-90 vol-%, preferably 55-95 vol-% or 55-90 vol-%, further preferably 60-95 vol-% or 60-90 vol-%, more preferably 70-95 vol-% or 70-90 vol-%, yet more preferably 75-95 vol-% or 75-90 vol-%, even more preferably 80-95 vol-% or 80-90 vol-%, and most preferably 85-95 vol-% or 85-90 vol-% of the total diesel fuel composition volume. The vol-% of renewable paraffinic diesel component in the diesel fuel composition may be selected so that the sum of the vol-% of di-GTBE and of the renewable paraffinic diesel component does not exceed 100 vol-%.

In certain embodiments, the combined amount of a. and b. in the diesel fuel composition is at least 95 vol-%, more preferably at least 97 vol-%, even more preferably at least 99 vol-% of the total diesel fuel composition volume. The beneficial lubricity properties, good compatibility with diesel engines and environmental sustainability of the diesel fuel composition are favored when the diesel fuel composition comprises to a large extent renewable paraffinic diesel component and di-GTBE.

In certain embodiments, the renewable paraffinic diesel component comprises paraffins at least 90 wt-%, preferably at least 95 wt-%, and more preferably at least 99 wt-% of the total weight of the renewable paraffinic diesel component.

In certain embodiments, the renewable paraffinic diesel component comprises paraffins in the range of carbon number C15-C18 at least 80 wt-%, preferably at least 90 wt-% of the total weight of paraffins in the renewable paraffinic diesel component.

In certain embodiments, the renewable paraffinic diesel component comprises i-paraffins and n-paraffins in a weight ratio of i-paraffins to n-paraffins of at least 2.2, at least 2,3, at least 3, or at least 4. Renewable paraffinic diesel components comprising i-paraffins and n-paraffins in a weight ratio of i-paraffins to n-paraffins of at least 2.2 provide the diesel fuel composition with beneficial cold properties. The cold properties improve as the weight ratio of i-paraffins to n-paraffins increases.

According to a second aspect of the invention, there is provided a method for
producing a diesel fuel composition, comprising:
providing a renewable paraffinic diesel component comprising;
   i. providing a feedstock originating from renewable sources, the feedstock comprising fatty acids, or triglycerides, or both;
   ii. subjecting the renewable feedstock to hydrotreatment, preferably hydrodeoxygenation, to produce n-paraffins; and optionally
   iii. subjecting at least a portion of the n-paraffins from step ii) to an isomerization treatment to produce i-paraffins;
providing di-glycerol tert-butyl ether (di-GTBE); and
mixing the renewable paraffinic diesel component with the di-GTBE to form a diesel fuel composition comprising at least 5 vol-% di-GTBE and at most 95 vol-% renewable paraffinic diesel component of the total diesel fuel composition volume.

Diesel fuel compositions produced with such methods have beneficial lubricity properties.

In certain embodiments, providing di-GTBE comprises:
I. providing glycerol from a waste stream or a side stream of fatty acid methyl esters (FAME) production; and
II. subjecting said glycerol to an etherification treatment with iso-butene derived from renewable sources to produce di-GTBE.

Providing di-GTBE originating from renewable sources (renewable di-GTBE) increases the percentage of renewable components in the formed diesel fuel composition and thus improves its environmental sustainability. Providing renewable di-GTBE enables production of a fully renewable diesel fuel composition.

The present diesel fuel composition may for example be formed by a method for forming a diesel fuel composition according to the first aspect, comprising:
mixing a renewable paraffinic diesel component with di-glycerol tert-butyl ether (di-GTBE) to form the diesel fuel composition according to the first aspect.

According to a third aspect of the invention there is provided use of a diesel fuel composition according to the first aspect as a fuel for a diesel engine. Use of the diesel fuel composition of the first aspect as a diesel engine fuel decreases the friction and wear on diesel engines during operation because of the beneficial lubricity properties of the diesel fuel composition.

According to a fourth aspect of the invention, there is provided use of di-glycerol tert-butyl ether (di-GTBE) as a lubricity improver for fuels. It has surprisingly been found that adding di-GTBE to fuels result in fuel compositions with enhanced lubricity properties compared to the neat fuels.

In certain embodiments, di-GTBE is used as a lubricity improver for renewable diesel fuels, preferably renewable paraffinic diesel fuels. Di-GTBE may be used as a sole lubricity improver for a renewable diesel fuel, i.e. other lubricity improvers are not necessarily needed.

In certain embodiments, di-GTBE is added to the fuel to form a combined fuel composition comprising at least 5 vol-%, preferably at least 10 vol-%, di-GTBE of the total combined fuel composition volume. Adding such amount of di-GTBE provides the formed combined fuel composition with beneficial lubricity properties.

In certain embodiments, di-GTBE is added to the fuel to form a combined fuel composition comprising 5-50 vol-% or 5-45 vol-%, preferably 5-40 vol-% or 5-30 vol-%, further preferably 5-25 vol-%, more preferably 5-20 vol-% di-GTBE of the total combined fuel composition volume. In certain embodiments, di-GTBE is added to the fuel to form a combined fuel composition comprising 5-22 vol-% or 5-18 vol-% di-GTBE, preferably 5-15 vol-% di-GTBE, further preferably 5-13 vol-% di-GTBE, more preferably 7-11 vol-% di-GTBE, even more preferably 8-10 vol-% di-GTBE of the total combined fuel composition volume. Adding such amounts of di-GTBE to the fuel results in a combined fuel composition with beneficial lubricity properties (HRFF-lubricity of 460 µm or less).

In certain embodiments, di-GTBE is added to the fuel to form a combined fuel composition comprising 10-50 vol-% or 10-45 vol-%, preferably 10-40 vol-%or 10-30 vol-%, further preferably 10-25 vol-%, more preferably 10-20 vol-% di-GTBE of the total combined fuel composition volume. In certain embodiments, di-GTBE is added to the fuel to form a combined fuel composition comprising 10-22 vol-% or 10-19 vol-% di-GTBE, preferably 10-17 vol-% di-GTBE, further preferably 10-15 vol-% di-GTBE, more preferably 10-12 vol-% di-GTBE of the total combined fuel composition volume. Adding such amounts of di-GTBE to the fuel results in a combined fuel composition with particularly good lubricity properties (HRFF-lubricity below 400 µm).

Herein is also described a method for decreasing High Frequency Reciprocating Rig (HFRR) -lubricity (measured according to EN ISO12156-1:2016) of a fuel, the method comprising adding di-GTBE to the fuel. It has been found that adding di-GTBE to a fuel results in a fuel composition with a lower HFRR-lubricity value than measured for the neat fuel.

For example, the method may comprise adding di-GTBE to the fuel to form a combined fuel composition comprising at least 5 vol-%, preferably at least 10 vol-% di-GTBE of the total combined fuel composition volume. For example, the method may comprise adding di-GTBE to the fuel to form a combined fuel composition comprising 5-50 vol-% or 5-45 vol-%, preferably 5-40 vol-% or 5-30 vol-%, further preferably 5-25 vol-%, more preferably 5-20 vol-% di-GTBE of the total combined fuel composition volume. Fo example, the method may comprise adding di-GTBE to the fuel to form a combined fuel composition comprising 10-50 vol-% or 10-45 vol-%, preferably 10-40 vol-% or 10-30 vol-%, further preferably 10-25 vol-%, more preferably 10-20 vol-% di-GTBE of the total combined fuel composition volume. For example, the method may comprise adding di-GTBE to the fuel to form a combined fuel composition comprising 5-22 vol-%, preferably 5-18 vol-% or 5-15 vol-%, further preferably 5-13 vol-%, more preferably 7-11 vol-%, even more preferably 8-10 vol-% di-GTBE of the total combined fuel composition volume. For example, the method may comprise adding di-GTBE to the fuel to form a combined fuel composition comprising 10-22 vol-% or 10-19 vol-%, preferably 10-17 vol-% or 10-15 vol-%, further preferably 10-13 vol-% or 10-12 vol-% di-GTBE of the total combined fuel composition volume. The benefits and effects of the examples hereabove are explained in connection with corresponding embodiments of the fourth aspect.

### BRIEF DESCRIPTION OF THE DRAWINGS

Some example embodiments of the invention will be described with reference to the accompanying drawings, in which:

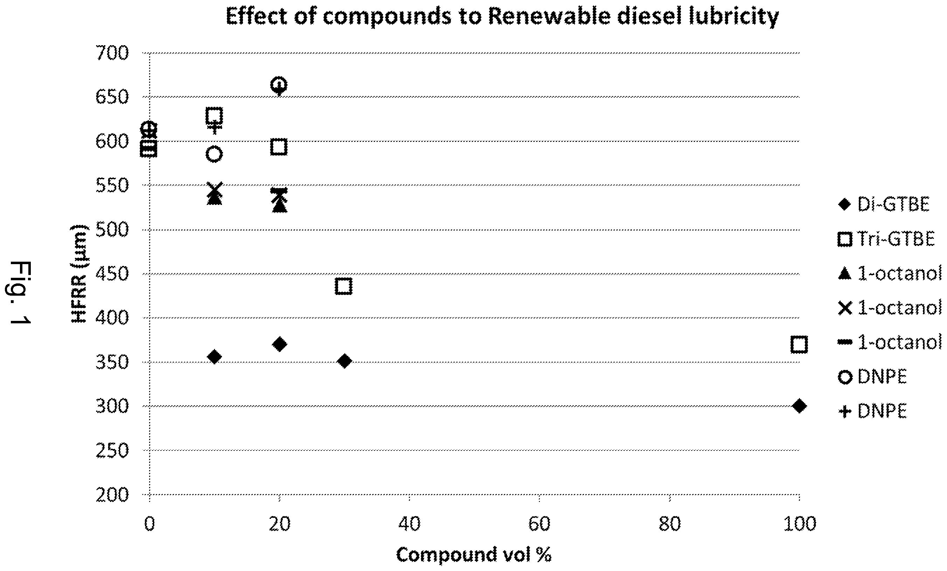

- Fig. 1: shows a graph presenting High Frequency Reciprocating Rig (HFRR) -lubricity values of blends comprising renewable paraffinic diesel component from one of three batches and one of 1-octanol, di-n-pentyl ether (DNPE), tri-glycerol tert-butyl ether (tri-GTBE), and di- glycerol tert-butyl ether (di-GTBE) as a second component. The HFRR-lubricity values are plotted as a function of vol-%, based on the total blend volumes, of the second component in said blends; wherein the diamonds denote blends of batch 1 paraffinic diesel component and di-GTBE, the squares denote blends of batch 1 paraffinic diesel component and tri-GTBE, the triangles denote blends of batch 3 paraffinic diesel component and 1-octanol, the x-symbols denote blends of batch 2 paraffinic diesel component and 1-octanol, the oblongs (rectangles) denote blends of batch 1 paraffinic diesel component and 1-octanol, the circles denote blends of batch 3 paraffinic diesel component and DNPE, and the crosses (+-symbols) denote blends of batch 2 paraffinic diesel component and DNPE.

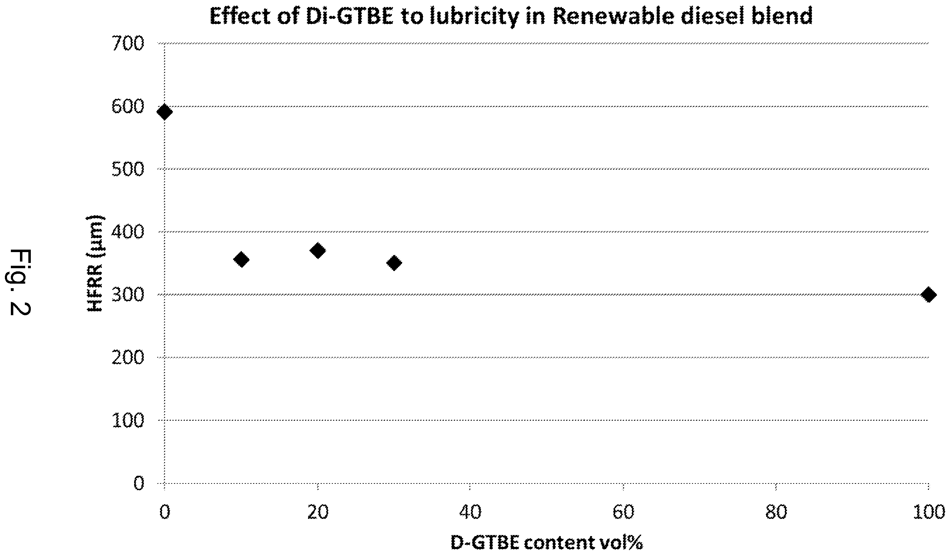

- Fig. 2: shows a graph presenting High Frequency Reciprocating Rig (HFRR) -lubricity values of blends comprising batch 1 renewable paraffinic diesel component and di-GTBE. The HFRR-lubricity values are plotted as a function of vol-%, based on the total blend volume, of di-GTBE;

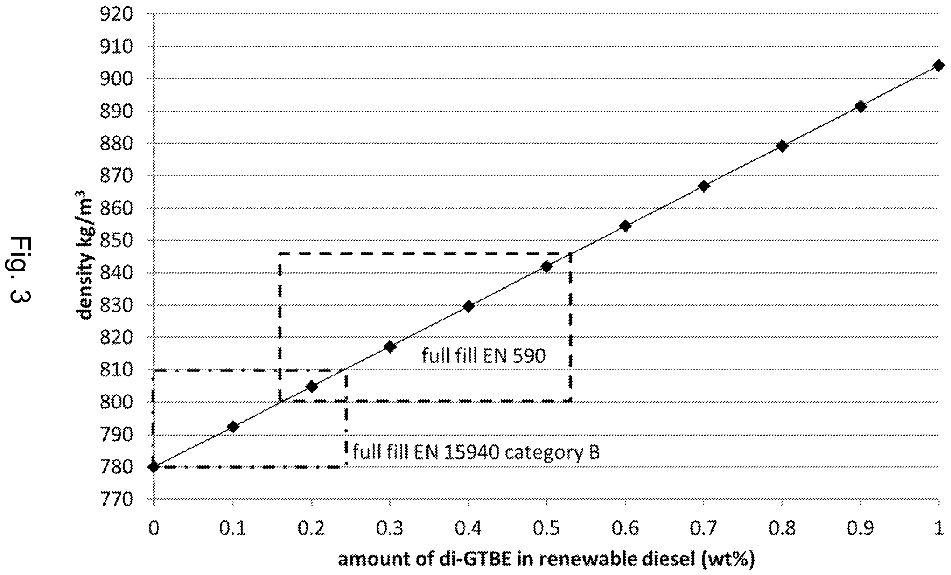

- Fig. 3: shows a graph presenting density of blends comprising a renewable paraffinic diesel and di-GTBE. The density values are plotted as a function of the wt-%, based on the total blend weight, of di-GTBE. The wt-% on the horizontal axis are denoted such that 1 denotes 100 wt-%, 0.9 denotes 90 wt-%, 0.8 denotes 80 wt-% etc.

### DETAILED DESCRIPTION

The present invention provides a diesel fuel composition, comprising a renewable paraffinic diesel component and di-glycerol tert-butyl ether (di-GTBE), and having beneficial lubricity properties. Beneficial lubricity properties refers herein to a low lubricity value of the diesel fuel composition (as measured according to EN ISO12156-1:2016), particularly lubricity values below 460 µm, which is the maximum lubricity value allowed by standard EN 590:2017 for automotive diesel fuel and standard EN 15940:2016 for automotive paraffinic diesel fuel from synthesis or hydrotreatment. Beneficial lubricity properties may herein also refer to lubricity values that are below the lubricity values of neat renewable paraffinic diesel components.

The diesel fuel composition comprises a renewable paraffinic diesel component and at least 5 vol-% di-GTBE of the total diesel fuel composition volume. It has surprisingly been found, that diesel fuel compositions comprising a renewable paraffinic diesel component and at least 5 vol-% di-GTBE of the total diesel fuel composition volume meet the lubricity requirement set by EN 590:2017 and EN 15940:2016, i.e. such diesel fuel compositions have a High Frequency Reciprocating Rig (HFRR) -lubricity, measured according to EN ISO12156-1:2016, of at most 460 µm.

The beneficial lubricity properties, compatibility with diesel engines, and environmental sustainability of the diesel fuel composition are favored when the diesel fuel composition comprises to a large extent renewable paraffinic diesel component and di-GTBE. Therefore, in certain embodiments, the diesel fuel composition comprises at least 5 vol-% di-GTBE of the total diesel fuel composition volume, the combined amount of the renewable paraffinic diesel component and di-GTBE in the diesel fuel composition being at least 95 vol-%, preferably at least 97 vol-%, more preferably at least 99 vol-% of the total diesel fuel composition volume.

In addition to the renewable paraffinic diesel component and di-GTBE, the diesel fuel composition may comprise diesel fuel additives known in the art, preferably less than 5 vol-%, further preferably less than 3 vol-%, and more preferably less than 1 vol-% of the total diesel fuel composition volume. Such diesel fuel additives are for example cold flow improvers and/or conductivity improvers. Diesel fuel additives may be added into the diesel fuel composition to enhance certain properties, such as stability, cold flow properties or cetane number. In certain preferred embodiments, the diesel fuel composition does not comprise any lubricity improver or lubricity enhancer other than di-GTBE. The combination of the renewable paraffinic diesel component and di-GTBE already provides the diesel fuel composition with satisfactory lubricity properties. Accordingly, further lubricity additization of the diesel fuel composition is not necessary. However, optionally and if desired, the diesel fuel composition may comprise diesel fuel lubricity improvers know in the art.

In certain embodiments, the diesel fuel composition comprises 5-50 vol-% di-GTBE and 50-95 vol-% renewable paraffinic diesel component, or 5-45 vol-% di-GTBE and 55-95 vol-% renewable paraffinic diesel component, preferably 5-40 vol-% di-GTBE and 60-95 vol-% renewable paraffinic diesel component or 5-30 vol-% di-GTBE and 70-95 vol-% renewable paraffinic diesel component, further preferably 5-25 vol-% di-GTBE and 70-95 vol-% renewable paraffinic diesel component, more preferably 5-20 vol-% di-GTBE and 80-95 vol-% renewable paraffinic diesel component of the total diesel fuel composition volume. Such diesel fuel compositions have good compatibility with automotive diesel engines and beneficial lubricity properties, the lubricity properties meeting the requirement of EN 590:2017 and EN 15940:2016. Further, such diesel fuel compositions have their densities within the range from 780 kg/m³ to 845kg/m³. Such densities fulfill the density requirements set for EN 15940:2016 category B diesel fuels and/or EN 590:2017 diesel fuels. The allowed density range is 780-810 kg/m³ for EN 15940:2016 category B diesel fuels, and 800-845 kg/m³ for EN 590:2017 diesel fuels. Hence, these diesel fuel compositions fulfill the requirements of EN 15940:2016 category B and/or EN 590:2017.

In certain embodiments, the diesel fuel composition comprises at least 10 vol-% di-GTB of the total diesel fuel composition volume. Diesel fuel compositions comprising a renewable paraffinic diesel component and at least 10 vol-% di-GTBE have been found to have a HFRR-lubricity, measured according to EN ISO12156-1:2016, of less than 400 µm, thus having particularly beneficial lubricity properties. HFRR-lubricities of less than 400 µm are significantly below the allowed maximum HFRR-lubricity of EN 590:2017 and EN 15940:2016. Further, in certain embodiments, the diesel fuel composition of the present invention comprises at least 10 vol-% di-GTB of the total diesel fuel composition volume, the combined amount of the renewable paraffinic diesel component and di-GTBE in the diesel fuel composition being at least 95 vol-%, preferably at least 97 vol-%, more preferably at least 99 vol-% of the total diesel fuel composition volume. The particularly beneficial lubricity properties, compatibility with diesel engines, and environmental sustainability of the diesel fuel composition are favored when the combined amount of the renewable paraffinic diesel component and di-GTBE in the diesel fuel composition is high (for example, at least 95 vol-%, or at least 97 vol-%, or at least 99 vol-%), or when the diesel fuel composition consists mainly of the renewable paraffinic diesel component and di-GTBE.

In certain embodiments, the diesel fuel composition of the present invention comprises 10-50 vol-% di-GTBE and 50-90 vol-% renewable paraffinic diesel component or 10-45 vol-% di-GTBE and 55-90 vol-% renewable paraffinic diesel component, preferably 10-40 vol-% di-GTBE and 60-90 vol-% renewable paraffinic diesel component or 10-30 vol-% di-GTBE and 70-90 vol-% renewable paraffinic diesel component, further preferably 10-25 vol-% di-GTBE and 75-90 vol-% renewable paraffinic diesel component, more preferably 10-20 vol-% di-GTBE and 80-90 vol-% renewable paraffinic diesel component of the total diesel fuel composition volume. Such diesel fuel compositions have good compatibility with automotive diesel engines and particularly beneficial lubricity properties, namely a HFRR-lubricity below 400 µm. Further, such diesel fuel compositions fulfill the requirements of EN 15940:2016 category B and/or EN 590:2017.

Typically, the density of the renewable paraffinic diesel component is within the density range of EN 15940:2016 (category B), but does not meet the 800 kg/m³ minimum density of EN 590:2017. In certain embodiments, the density of the renewable paraffinic diesel component is approximately 780 kg/m³ (at 15 °C). The density of neat di-GTBE (at 15 °C) is 904 kg/m³ which exceeds the allowed maximum densities of both EN 15940:2016 (category B) and EN 590:2017, namely 810 kg/m³ and 845 kg/m³ respectively. It has been found that diesel fuel compositions of the first aspect comprising at most 22 vol-% di-GTBE and at least 78 vol-% renewable paraffinic diesel component fulfill the density requirements of EN 15940:2016 category B. Further, it has been found that diesel fuel compositions of the first aspect comprising 13-51 vol-% di-GTBE and 49-87 vol-% renewable paraffinic diesel component fulfill the density requirements of EN 590:2017. Thus, diesel fuel compositions of the first aspect comprising 13-22 vol-% di-GTBE and 78-87 vol-% renewable paraffinic diesel component fulfill the density requirements of both EN 15940:2016 (category B) and EN 590:2017.

Surprisingly, it has been found that the HFRR-lubricity of the diesel fuel composition according to the present invention does not decrease linearly when increasing the vol-% of di-GBTE in the diesel fuel composition. After dropping significantly when increasing the vol-% of di-GTBE in the diesel fuel composition from 0 vol-% to approximately 10 vol-%, the HFRR-lubricity decreases more modestly when the amount of di-GBTE is increased to larger vol-% (vol-% above 10). Thus, in certain embodiments, the diesel fuel composition of the present invention comprises 5-22 vol-% di-GTBE and 78-95 vol-% renewable paraffinic diesel component, or 5-18 vol-% di-GTBE and 82-95 vol-% renewable paraffinic diesel component, preferably 5-15 vol-% di-GTBE and 85-95 vol-% renewable paraffinic diesel component, further preferably 5-13 vol-% di-GTBE and 87-95 vol-% renewable paraffinic diesel component, more preferably 7-11 vol-% di-GTBE and 89-93 vol-% renewable paraffinic diesel component, even more preferably 8-10 vol-% di-GTBE and 90-92 vol-% renewable paraffinic diesel component of the total diesel fuel composition volume. Surprisingly, such diesel fuel compositions whit a rather small vol-% of di-GTBE meet the lubricity requirement set by EN 590:2017 and EN 15940:2016. Further, such rather small vol-% of di-GTBE in the diesel fuel composition does not excessively affect the density of the diesel fuel composition keeping the density of the diesel fuel composition within the allowed density range of standard EN 15940:2016 (category B). Thus, such diesel fuel compositions fulfill the requirements of EN 15940:2016 (category B). A rather high vol-% of the renewable paraffinic diesel component is preferred, since it favors good performance of the diesel fuel composition as an automotive diesel fuel.

Further, in certain preferred embodiments, the diesel fuel composition of the present invention comprises 10-22 vol-% di-GTBE and 78-90 vol-% renewable paraffinic diesel component, or 10-19 vol-% di-GTBE and 81-90 vol-% renewable paraffinic diesel component, preferably 10-17 vol-% di-GTBE and 83-90 vol-% renewable paraffinic diesel component, further preferably 10-15 vol-% di-GTBE and 85-90 vol-% renewable paraffinic diesel component, more preferably 10-12 vol-% di-GTBE and 88-90 vol-% renewable paraffinic diesel component of the total diesel fuel composition volume. Surprisingly, such diesel fuel compositions have particularly beneficial lubricity properties, i.e. HFRR-lubricities below 400 µm, achieved with a moderate vol-% amount of di-GTBE in the diesel fuel composition. Further, such diesel fuel compositions fulfill the requirements of current diesel fuel standard, namely EN 15940:2016 (category B) and/or EN 590:2017.A rather high vol-% of the renewable paraffinic diesel component is preferred, since it favors good performance of the diesel fuel composition as an automotive diesel fuel.

Diesel fuel compositions comprising mainly the renewable paraffinic diesel component and di-GTBE are preferred. Such diesel fuel compositions perform very well as automotive diesel fuels, have beneficial lubricity properties, and meet the requirements of current diesel fuel standards (EN 15940:2016 (category B) and/or EN 590). These effects are favored as the combined amount of the renewable paraffinic diesel component and di-GTBE in the diesel fuel composition increases. Therefore, in any of the embodiments disclosed herein, the combined amount of the renewable paraffinic diesel component, and di-GTBE may be at least 95 vol-%, preferably at least 97 vol-%, and more preferably at least 99 vol-% of the total diesel fuel composition volume.

For example, in certain embodiments, the diesel fuel composition comprises di-GTBE 5-50 vol-%, or 5-40 vol-%, preferably 5-30 vol-%, further preferably 5-25 vol-%, more preferably 5-20 vol-%, and even more preferably 5-15 vol-% of the total diesel fuel composition volume, the combined amount of di-GTBE and the renewable paraffinic diesel component being at least 95 vol-% of the total diesel fuel composition volume. In certain embodiments, the diesel fuel composition comprises di-GTBE 5-50 vol-%, or 5-40 vol-%, preferably 5-30 vol-%, further preferably 5-25 vol-%, more preferably 5-20 vol-%, and even more preferably 5-15 vol-% of the total diesel fuel composition volume, the combined amount of di-GTBE and the renewable paraffinic diesel component being at least 97 vol-% of the total diesel fuel composition volume. In certain embodiments, the diesel fuel composition comprises di-GTBE 5-50 vol-%, or 5-40 vol-%, preferably 5-30 vol-%, further preferably 5-25 vol-%, more preferably 5-20 vol-%, and even more preferably 5-15 vol-% of the total diesel fuel composition volume, the combined amount of di-GTBE and the renewable paraffinic diesel component being at least 99 vol-% of the total diesel fuel composition volume.

Further, in certain embodiments, the diesel fuel composition comprises di-GTBE 5-22 vol-%, or 5-18 vol-%, preferably 5-15 vol-%, further preferably 5-13 vol-%, more preferably 7-11 vol-%, even more preferably 8-10 vol-% of the total diesel fuel composition volume, the combined amount of di-GTBE and the renewable paraffinic diesel component being at least 97 vol-% of the total diesel fuel composition volume. In certain embodiments, the diesel fuel composition comprises di-GTBE 5-22 vol-%, or 5-18 vol-%, preferably 5-15 vol-%, further preferably 5-13 vol-%, more preferably 7-11 vol-%, even more preferably 8-10 vol-% of the total diesel fuel composition volume, the combined amount of di-GTBE and the renewable paraffinic diesel component being at least 99 vol-% of the total diesel fuel composition volume.

Yet further, in certain embodiments, the diesel fuel composition comprises di-GTBE 10-50 vol-%, or 10-40 vol-%, preferably 10-30 vol-%, further preferably 10-25 vol-%, more preferably 10-20 vol-%, and even more preferably 10-15 vol-% of the total diesel fuel composition volume, the combined amount of di-GTBE and the renewable paraffinic diesel component being at least 95 vol-% of the total diesel fuel composition volume. In certain embodiments, the diesel fuel composition comprises di-GTBE 10-50 vol-%, or 10-40 vol-%, preferably 10-30 vol-%, further preferably 10-25 vol-%, more preferably 10-20 vol-%, and even more preferably 10-15 vol-% of the total diesel fuel composition volume, the combined amount of di-GTBE and the renewable paraffinic diesel component being at least 97 vol-% of the total diesel fuel composition volume. In certain embodiments, the diesel fuel composition comprises di-GTBE 10-50 vol-%, or 10-40 vol-%, preferably 10-30 vol-%, further preferably 10-25 vol-%, more preferably 10-20 vol-%, and even more preferably 10-15 vol-% of the total diesel fuel composition volume, the combined amount of di-GTBE and the renewable paraffinic diesel component being at least 99 vol-% of the total diesel fuel composition volume.

Still further, in certain embodiments, the diesel fuel composition comprises di-GTBE 10-22 vol-%, or 10-19 vol-%, preferably 10-17 vol-%, further preferably 10-15 vol-%, more preferably 10-13 vol-% or 10-12 vol-% of the total diesel fuel composition volume, the combined amount of di-GTBE and the renewable paraffinic diesel component being at least 97 vol-% of the total diesel fuel composition volume. In certain embodiments, the diesel fuel composition comprises di-GTBE 10-22 vol-%, or 10-19 vol-%, preferably 10-17 vol-%, further preferably 10-15 vol-%, more preferably 10-13 vol-% or 10-12 vol-% of the total diesel fuel composition volume, the combined amount of di-GTBE and the renewable paraffinic diesel component being at least 99 vol-% of the total diesel fuel composition volume.

In certain embodiments, the diesel fuel composition comprises 13-22 vol-% di-GTBE, the combined amount of di-GTBE and the renewable paraffinic diesel component being at least 95 vol-%, preferably at least 97 vol-% and more preferably at least 99 vol-% of the total diesel fuel composition volume. Such diesel fuel compositions have particularly beneficial lubricity properties and fulfill the requirements of both EN 15940:2016 (category B) and EN 590:2017.

The renewable paraffinic diesel component comprised in the diesel fuel composition of the present invention is a component derived from a renewable source or renewable sources and comprising to a large extent paraffins (alkanes), and is suitable for use as diesel engine fuel as such, or, for example, after additization with known diesel fuel additives, such as cold flow improvers and/or conductivity improvers. Preferably, the renewable paraffinic diesel component is suitable for use as a fuel for automotive diesel engines. The renewable paraffinic diesel components may also be referred to as "bio-based paraffinic diesel component", "hydrotreated vegetable oil fuel", "hydrotreated vegetable oil", "hydrotreated renewable diesel", "renewable fuel", "renewable diesel", or "renewable diesel component".

Components or compositions derived from renewable (bio-based) sources have a higher content of ¹⁴C isotopes than corresponding components or compositions derived from fossil (fossil based) sources. Said higher content of ¹⁴C isotopes is an inherent feature of renewable components or compositions owing to the starting material, i.e. the renewable sources, from which they are derived. Carbon atoms of renewable origin comprise a higher number of ¹⁴C isotopes compared to carbon atoms of fossil origin. The isotope ratio of renewable carbon does not change in the course of chemical reactions. It is thus possible to distinguish between a carbon compound or composition derived from renewable sources and carbon compounds derived from fossil sources by analysing the ratio of ¹²C and ¹⁴C isotopes. The ¹⁴C isotope content of a compound or composition can be measured and quantified by standard methods, such as ASTM D 6866 or DIN 51637. Typically, in a component or composition derived completely from renewable sources the measured ¹⁴C content of the total carbon content in the component or composition is at least 100% (± measurement accuracy). The amount of renewable carbon in the composition can thus be quantified based on the ¹⁴C isotope profile, and be used to determine the nature and origin of its components. The nature and origin of diesel components and diesel fuels can thus be confirmed and distinguished by ¹⁴C analysis.

In certain embodiments, the renewable paraffinic diesel component is a renewable paraffinic diesel fuel. Preferably, the renewable paraffinic diesel component is a low-sulfur diesel component comprising sulfur less than 5 ppm by weigh (parts per million by weight), or less than 5 mg sulfur/kg renewable paraffinic diesel component. Such low sulfur diesel components burn cleaner compared to components with a higher sulfur content. This reduces harmful emissions.

The renewable paraffinic diesel component is preferably derived from renewable sources via hydrotreatment optionally followed by an isomerization treatment. The renewable paraffinic diesel component is thus preferably a paraffinic diesel fuel from hydrotreatment. Preferably, the renewable sources from which the renewable paraffinic diesel component is derived are renewable oils, renewable fats, or a combination thereof. Chemically hydrotreated renewable oils and/or fats are mixtures of mainly linear paraffinic hydrocarbons (normal paraffins, n-paraffins) comprising a very low quantity of sulfur and aromatics. Renewable paraffinic diesel components obtained from hydrotreatment followed by isomerization can be used as the renewable paraffinic diesel component or as renewable paraffinic diesel fuel in any aspect and embodiment of the present invention. Hydrotreatment and optional isomerization of renewable oils and/or fats typically produces bio-based middle distillate fuels.

In certain embodiments, the renewable paraffinic diesel component is a renewable middle distillate fuel having a boiling point range (initial boiling point to end point) as measured according to EN-ISO-3405 (2011) within the temperature range 180-360 °C, preferably 180-320 °C, further preferably 200-300 °C, and more preferably 250-300 °C. These renewable middle distillate fuel fractions perform particularly well as diesel fuels.

In certain embodiments, the renewable paraffinic diesel component comprises at least 90 wt-%, preferably at least 95 wt-%, more preferably at least 99 wt-% paraffins of the total weight of the renewable paraffinic diesel component. The renewable paraffinic diesel component may comprise at least 91 wt-%, 92 wt-%, 93 wt-%, 94 wt-%, 96 wt-%, 97wt-%, or 98 wt-% paraffins based on the total weight of the renewable paraffinic diesel component. A high paraffin content of the renewable paraffinic diesel component is preferred, because upon combustion paraffins form less soot, polycyclic aromatic hydrocarbons (PAH) and NOₓ compounds compared to combustion of aromatic compounds. In certain embodiments, the renewable paraffinic diesel component has a low olefin (alkene) content and comprises less than 2.0 wt-%, preferably at most 1.0 wt-%, and more preferably at most 0.5 wt-% olefins of the total weight of the renewable paraffinic diesel component. Further, in certain preferred embodiments, the renewable paraffinic diesel component has a low content of naphthenes (cycloalkanes) and comprises at most 5.0 wt-%, preferably at most 2.0 wt-% naphthenes of the total weight of the renewable paraffinic diesel component.

Preferably, the renewable paraffinic diesel component has a low content, or is free from, aromatic compounds (aromatics). Accordingly, in certain embodiments, the renewable paraffinic diesel component comprises at most 1.0 wt-%, preferably at most 0.5 wt-%, more preferably at most 0.2 wt-% aromatics of the total weight of the renewable paraffinic diesel component. Diesel components with a low aromatics content burn cleaner compared to components comprising more aromatic compounds thus reducing soot emissions. In certain embodiments, the renewable paraffinic diesel component comprises less than 5 mg sulfur/kg renewable paraffinic diesel component (5 ppm by weight) and at most 1.0 wt-%, preferably at most 0.5 wt-%, more preferably at most 0.2 wt-% aromatics of the total weight of the renewable paraffinic diesel component. Such renewable paraffinic diesel components burn particularly cleanly reducing harmful emissions. When blending renewable paraffinic diesel component having a low content of aromatics and/or sulfur with di-GTBE, the resulting diesel fuel composition also has a low content of aromatics and/or sulfur and the associated benefits.

In certain embodiments, the renewable paraffinic diesel component comprises at least 95 wt-% paraffins, at most 1.0 wt-% olefins, at most 0.5 wt-% aromatics, and at most 2.0 wt-% naphthenes based on the total weight of the renewable paraffinic diesel component. Further, in certain embodiments, the renewable paraffinic diesel component comprises at least 97 wt-% paraffins, at most 0.5 wt-% olefins, at most 0.2 wt-% aromatics, and at most 2.0 wt-% naphthenes based on of the total weight of the renewable paraffinic diesel component. Such renewable paraffinic diesel components perform particularly well as diesel fuel components. The renewable paraffinic diesel component comprises preferably mainly hydrocarbons, and accordingly, in certain embodiments, the renewable paraffinic diesel component contains at most 1 wt-% oxygen based on all elements constituting the renewable paraffinic diesel component as determined by elemental analysis. Such renewable diesel fuel components have beneficial oxidation stability and storage properties.

The renewable paraffinic diesel component of the invention comprises at least 70 wt-%, preferably at least 80 wt-%, such as at least 88 wt-%, more preferably at least 90 wt-% paraffins in the range of carbon number C15-C18 of the total weight of the paraffins in the renewable paraffinic diesel component. Optionally, in certain embodiments, the renewable paraffinic diesel component comprises paraffins in the range of carbon number C3-C14 less than 25 wt-%, such as less than 20 wt-%, or less than 10% wt-%, or preferable less than 7 wt-% of the total weight of paraffins in the renewable paraffinic diesel component. Optionally, in certain embodiments, the renewable paraffinic diesel component comprises paraffins in the range of carbon number C19-C24 less than 25 wt- %, such as less than 20 wt-%, or less than 10 wt-%, preferably less than 5 wt-% of the total weight of paraffins in the renewable paraffinic diesel component.

The above carbon number distributions are typical for renewable paraffinic diesel components derived through hydrotreatment of renewable oils and/or fats. Fuel components manufactured through gas-to-liquid (GTL) processes, such as processes comprising a Fischer-Tropsch process step, have a much broader distribution of paraffinic hydrocarbons (paraffins) compared to the above-described carbon number distributions. GTL-fuels are characterized by broad distribution of paraffinic hydrocarbons in the range C9-C24. In certain embodiments, the renewable paraffinic diesel component is obtained without GTL-process.

In certain embodiments, the renewable paraffinic diesel component comprises at least 95 wt-% paraffins of the total weight of the renewable paraffinic diesel component, and at least 70 wt-%, preferably at least 80 wt-%, more preferably at least 90 wt-%, of said paraffins are in the range of carbon number C15-C18 based on the total weight of paraffins in the renewable paraffinic diesel component. Further, in certain preferred embodiments, the renewable paraffinic diesel component comprises at least 99 wt-% paraffins of the total weight of the renewable paraffinic diesel component, and at least 70 wt-%, preferably at least 80 wt-%, more preferably at least 90 wt-%, of said paraffins are in the range of carbon number C15-C18 based on the total weight of paraffins in the renewable paraffinic diesel component. Such renewable paraffinic diesel components have predictable properties and perform well as diesel fuel components.

In certain embodiments, the paraffins of the renewable paraffinic diesel component comprises both isoparaffins (i-paraffins) and normal paraffins (n-paraffins). Isoparaffins improve the cold properties, i.e. lower the cloud point or the pour point, of the renewable paraffinic diesel component and consequently of the diesel fuel composition. A weight ratio of i-paraffins to n-paraffins in the renewable paraffinic diesel component of at least 2.2 yields a renewable paraffinic diesel component having a pour point below 0 °C (as measured according to ASTM D5950-14). Accordingly, in certain embodiments, the weight ratio of i-paraffins to n-paraffins in the renewable paraffinic diesel component is at least 2.2. Increasing the weight ratio of i-paraffins to n-paraffins further improves the cold properties of the renewable paraffinic diesel component. In certain embodiments, the weight ratio of i-paraffins to n-paraffins in the renewable paraffinic diesel component is at least 2.3, at least 3, or at least 4. A weight ratio of i-paraffins to n-paraffins in the renewable paraffinic diesel component of at least 4 yields a renewable paraffinic diesel component having a cloud point of approximately -20 °C or below. The weight ratio of i-paraffins to n-paraffins in the renewable paraffinic diesel component may be about 2.2, 2.3, 2.4, 2.5, 2.6, 10 2.7, 2.8, 2.9, 3, 4, 5, 6, 7, 8, 9 or 10. A renewable paraffinic diesel component comprising both n-paraffins and i-paraffins can be used in any aspect and embodiment of the present invention. In certain embodiments, the renewable paraffinic diesel component comprises at least 95 wt-% paraffins of the total weight of the renewable paraffinic diesel component, the weight ratio of i-paraffins to n-paraffins of said paraffins being at least 2.3, at least 3, or at least 4. Further, in certain embodiments, he renewable paraffinic diesel component comprises at least 99 wt-% paraffins of the total weight of the renewable paraffinic diesel component, the weight ratio of i-paraffins to n-paraffins of said paraffins being at least 2.3, at least 3, or at least 4.

In certain embodiments, the renewable paraffinic diesel component comprises at least 90 wt-%, preferably at least 95 wt-%, more preferably at least 99 wt-% paraffins of the total weight of the renewable paraffinic diesel component, and of said paraffins at least 80 wt-%, based on the total weight paraffins in the renewable paraffinic diesel component, are in the in the range of carbon number C15-C18, and the weight ratio of i-paraffins to n-paraffins in the renewable paraffinic diesel component is at least 2.2. Further, in certain embodiments, the renewable paraffinic diesel component comprises at least 90 wt-%, preferably at least 95 wt-%, more preferably at least 99 wt-% paraffins, and of said paraffins, based on the total weight of paraffins in the renewable paraffinic diesel component, at least 90 wt-% are in the in the range of carbon number C15-C18, and the weight ratio of i-paraffins to n-paraffins in the renewable paraffinic diesel component is at least 2.2. Such renewable paraffinic diesel components are particularly preferred in diesel fuel compositions with di-GTBE.

The renewable paraffinic diesel component described in the foregoing may be provided as the renewable paraffinic diesel component or renewable paraffinic diesel fuel in any aspect and embodiment of the present invention.

In certain embodiments, the diesel fuel composition comprises at least 5 vol-%, preferably at least 10 vol-% di-GTBE of the total diesel fuel composition volume, the combined amount of di-GTBE and the renewable paraffinic diesel component being at least 95 vol-% of the total diesel fuel composition volume, and wherein the renewable paraffinic diesel component comprises at least 90 wt-% paraffins of the renewable paraffinic diesel component, of said paraffins of the renewable paraffinic diesel component at least 70 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. Further, in certain embodiments, the diesel fuel composition comprises at least 5 vol-%, preferably at least 10 vol-% di-GTBE of the total diesel fuel composition volume, the combined amount of di-GTBE and the renewable paraffinic diesel component being at least 97 vol-% of the total diesel fuel composition volume, and wherein the renewable paraffinic diesel component comprises at least 99 wt-% paraffins of the renewable paraffinic diesel component, of said paraffins of the renewable paraffinic fuel component at least 90 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. These diesel fuel compositions are particularly preferred.

Further, in certain embodiments, the diesel fuel composition comprises 5-50 vol-% di-GTBE and 50-95 vol-% renewable paraffinic diesel component, or 5-40 vol-% di-GTBE and 60-95 vol-% renewable paraffinic diesel component, preferably 5-20 vol-% di-GTBE and 80-95 vol-% renewable paraffinic diesel component, further preferably 10-20 vol-% di-GTBE and 80-90 vol-% renewable paraffinic diesel component, and more preferably 10-15 vol-% di-GTBE and 85-90 vol-% renewable paraffinic diesel component of the total diesel fuel composition volume, the combined amount of di-GTBE and the renewable paraffinic diesel component preferably being at least 95 vol-% of the total diesel fuel composition volume, and wherein the renewable paraffinic diesel component comprises at least 90 wt-% paraffins of the renewable paraffinic diesel component, of said paraffins of the renewable paraffinic diesel component at least 70 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. Further, in certain embodiments, the diesel fuel composition comprises 5-50 vol-% di-GTBE and 50-95 vol-% renewable paraffinic diesel component, or 5-40 vol-% di-GTBE and 60-95 vol-% renewable paraffinic diesel component, preferably 5-20 vol-% di-GTBE and 80-95 vol-% renewable paraffinic diesel component, further preferably 10-20 vol-% di-GTBE and 80-90 vol-% renewable paraffinic diesel component, and more preferably 10-15 vol-% di-GTBE and 85-90 vol-% renewable paraffinic diesel component of the total diesel fuel composition volume, the combined amount of di-GTBE and the renewable paraffinic diesel component preferably being at least 95 vol-% of the total diesel fuel composition volume, and wherein the renewable paraffinic diesel component comprises at least 95 wt-% paraffins of the renewable paraffinic diesel component, of said paraffins of the renewable paraffinic diesel component at least 80 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. Yet further, in certain embodiments, the diesel fuel composition comprises 5-50 vol-% di-GTBE and 50-95 vol-% renewable paraffinic diesel component, or 5-40 vol-% di-GTBE and 60-95 vol-% renewable paraffinic diesel component, preferably 5-20 vol-% di-GTBE and 80-95 vol-% renewable paraffinic diesel component, further preferably 10-20 vol-% di-GTBE and 80-90 vol-% renewable paraffinic diesel component, and more preferably 10-15 vol-% di-GTBE and 85-90 vol-% renewable paraffinic diesel component of the total diesel fuel composition volume, the combined amount of di-GTBE and the renewable paraffinic diesel component preferably being at least 95 vol-% of the total diesel fuel composition volume, and wherein the renewable paraffinic diesel component comprises at least 99 wt-% paraffins of the renewable paraffinic diesel component, of said paraffins of the renewable paraffinic diesel component at least 90 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. These diesel fuel compositions are particularly preferred, due to their beneficial lubricity properties and beneficial diesel fuel properties. These diesel fuel compositions fulfill the requirements of EN 590:2017 and/or EN 15940:2016 (category B).

In certain embodiments, the diesel fuel composition comprises 13-22 vol-% di-GTBE, and 78-87 vol-% renewable paraffinic diesel component, wherein the renewable paraffinic diesel component comprises at least 90 wt-%, preferably at least 95 wt-%, further preferably at least 99 wt-% paraffins of the renewable paraffinic diesel component, of said paraffins of the renewable paraffinic diesel component at least 80 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. Further, in certain embodiments, the diesel fuel composition comprises 13-22 vol-% di-GTBE, and 78-87 vol-% renewable paraffinic diesel component, wherein the renewable paraffinic diesel component comprises at least 90 wt-%, preferably at least 95 wt-%, further preferably at least 99 wt-% paraffins of the renewable paraffinic diesel component, of said paraffins of the renewable paraffinic diesel component at least 90 wt-%, based on the total weight of paraffins in the renewable paraffinic diesel component, are in the range of carbon numbers C15-C18, the weight ratio of i-paraffins to n-paraffins in the renewable paraffinic diesel component being at least 2.2. These diesel fuel compositions are particularly preferred, due to their particularly beneficial lubricity properties and compliance with both EN 590:2017 and EN 15940:2016 (category B).

The present invention provides a method for producing a diesel fuel composition, the method comprising providing a renewable paraffinic diesel component, providing di-glycerol tert-butyl ether (di-GTBE), and mixing the renewable paraffinic diesel component with the di-GTBE to form a diesel fuel composition comprising at least 5 vol-% di-GTBE and at most 95 vol-% renewable paraffinic diesel component of the total diesel fuel composition volume. Diesel fuel compositions formed or manufactured according to this method have beneficial lubricity properties, namely HFRR -lubricity according to EN ISO12156-1:2016 of at most 460 µm. In certain embodiments, the method comprises mixing the renewable paraffinic diesel component with the di-GTBE to form a diesel fuel composition comprising, based on the total diesel fuel composition, at least 5 vol-% di-GTBE and at least 95 vol-%, preferably at least 97 vol-%, more preferably at least 99 vol-% di-GTBE and renewable paraffinic diesel component combined.

In certain embodiments, the method comprises mixing the renewable paraffinic diesel component with the di-GTBE to form a diesel fuel composition comprising at least 10 vol-% di-GTBE and at most 90 vol-% renewable paraffinic diesel component of the total diesel fuel composition volume. Diesel fuel compositions so produced have particularly beneficial lubricity properties, namely HFRR-lubricity according to EN ISO12156-1:2016 of less than 400 µm. In certain embodiments, the method of the second aspect comprises mixing the renewable paraffinic diesel component with the di-GTBE to form a diesel fuel composition comprising, based on the total diesel fuel composition, at least 10 vol-% di-GTBE and at least 95 vol-%, preferably at least 97 vol-%, more preferably at least 99 vol-% di-GBTBE and renewable paraffinic diesel component combined.

In certain embodiments, the method of the second aspect comprises mixing the renewable paraffinic diesel component with the di-GTBE to form a diesel fuel composition comprising 5-50 vol-% di-GTBE and 50-95 vol-% renewable paraffinic diesel component, or 5-40 vol-% di-GTBE and 60-95 vol-% renewable paraffinic diesel component, preferably 5-30 vol-% di-GTBE and 70-95 vol-% renewable paraffinic diesel component, further preferably 5-25 vol-% di-GTBE and 75-95 vol-% renewable paraffinic diesel component, more preferably 5-20 vol-% di-GTBE and 80-95 vol-% renewable paraffinic diesel component, and even more preferably 5-15 vol-% di-GTBE and 85-95 vol-% renewable paraffinic diesel component of the total diesel fuel composition volume.

In certain embodiments, method of the second aspect comprises mixing the renewable paraffinic diesel component with the di-GTBE to form a diesel fuel composition comprising 10-50 vol-% di-GTBE and 50-90 vol-% renewable paraffinic diesel component, or 10-40 vol-% di-GTBE and 60-90 vol-% renewable paraffinic diesel component, preferably 10-30 vol-% di-GTBE and 70-90 vol-% renewable paraffinic diesel component, further preferably 10-25 vol-% di-GTBE and 75-90 vol-% renewable paraffinic diesel component, more preferably 10-20 vol-% di-GTBE and 80-90 vol-% renewable paraffinic diesel component, and even more preferably 10-15 vol-% di-GTBE and 85-90 vol-% renewable paraffinic diesel component of the total diesel fuel composition volume.

In certain embodiments, method of the second aspect comprises mixing the renewable paraffinic diesel component with the di-GTBE to form a diesel fuel composition comprising 13-22 vol-% di-GTBE, and 78-87 vol-% renewable paraffinic diesel component of the total diesel fuel composition volume.

Providing a renewable paraffinic diesel component comprises providing a feedstock originating from renewable sources (renewable feedstock), the feedstock comprising fatty acids, or fatty acid derivatives, or mono-, di- or triglycerides, or a combination thereof, subjecting said renewable feedstockto hydrotreatment to produce n-paraffins, and optionally subjecting at least a portion of the n-paraffins from the hydrotreatment step to an isomerization treatment to produce i-paraffins. Renewable paraffinic diesel components provided through hydrotreatment and optional isomerization of renewable feedstock are particularly suitable for blending with di-GBTE to form diesel fuel compositions having good compatibility with diesel engines. Subjecting at least a portion of the n-paraffins to an isomerization treatment may comprise separating a portion of the n-paraffins from the hydrotreatment step, subjecting said portion to the isomerization treatment to form i-paraffins and optionally, after the isomerization treatment, reunifying the separated portion with the n-paraffins it was separated from.

Alternatively, the isomerization step may comprise subjecting all n-paraffins from the hydrotreatment step to an isomerization treatment. In certain embodiments, the hydrotreatment step and the optional isomerization step are conducted in separate reactors, or carried out separately. Optionally, the step of providing the renewable paraffinic diesel component may comprise a purification step(s) and/or a fractionation step(s). Purification and/or fractionation steps allows better control of the properties of the renewable paraffinic diesel component, and thus of the properties of the diesel fuel composition. In certain embodiments, providing the renewable paraffinic diesel component does not comprise gasifying renewable feedstock.

In certain embodiments, the hydrotreatment is performed at a pressure selected from, or varying within, the range 2-15 MPa, preferably 3-10 MPa, and at a temperature selected from, or varying within, the range 200-500 °C, preferably 280-400 °C. The hydrotreatment may be performed in the presence of known hydrotreatment catalysts containing metals from Group VIII and/or VIB of the Periodic System. Preferably, the hydrotreatment catalysts are supported Pd, Pt, Ni, NiW, NiMo or a CoMo catalyst, wherein the support is alumina and/or silica. Typically, NiMo/Al₂O₃ and/or CoMo/Al₂O₃ catalysts are used. Preferably, the hydrotreatment is hydrodeoxygenation (HDO), or catalytic hydrodeoxygenation (catalytic HDO).

The hydrotreatment typically serves as a deoxygenation, denitrogenation, and desulfurization treatment of the fatty acids, fatty acid derivatives, and/or the glycerides comprised in the renewable feedstock. Further, providing the renewable paraffinic diesel component may comprise subjecting the renewable feedstock to decarboxylation and decarbonylation reactions (i.e. removal of oxygen in the form of COₓ), and/or other catalytic processes to remove oxygen from organic oxygen compounds in the form of water, to remove sulfur from organic sulfur compounds in the form of dihydrogen sulfide (H₂S), to remove nitrogen from organic nitrogen compounds in the form of ammonia (NH₃) or to remove halogens from organic halogen compounds, for example chlorine in the form of hydrochloric acid (HCl). Such processes may be for example hydrodechlorination to remove chlorine and hydrodenitrogenation (HDN) to remove nitrogen.

In certain embodiments, n-paraffins from the hydrotreatment step may be subjected to catalytic cracking (CC), the catalytic cracking step then optionally followed by the isomerization treatment. CC allows adjusting the chain length of the paraffins. Typically, the carbon number distribution does not substantially change during the optional isomerization treatment. Therefore, in certain embodiments, the amount of paraffins in the carbon number range C3-C14 does not substantially increase during the optional isomerization treatment. Hence, the carbon number distribution of paraffins in the renewable paraffinic diesel component can be controlled.

In certain embodiments, providing the renewable paraffinic diesel component comprises subjecting at least a portion of the n-paraffins from the hydrotreatment step to the isomerization treatment to form i-paraffins. The isomerization treatment may be a catalytic isomerization treatment, such as hydroisomerization. Subjecting at least a portion of the renewable feedstock to the isomerization treatment increases the amount of isoparaffins in the (provided) renewable paraffinic diesel component. Typically, n-paraffins from the hydrotreatment step subjected to the isomerization treatment form i-paraffins having predominantly methyl branches. Accordingly, in certain embodiments, the i-paraffins from the isomerization step comprise one or more methyl branches. The severity of the isomerization conditions and the choice of catalyst controls the amount of methyl branches formed in the treatment and their distance from each other.

In certain embodiments, the isomerization treatment is performed at a temperature selected from, or varying within, the range 200-500°C, preferably 280-400°C, and at a pressure selected from, or varying within, the range 2-15 MPa, preferably 3-10 MPa. The isomerization treatment may be performed in the presence of known isomerization catalysts, for example catalysts containing a molecular sieve and/or a metal selected from Group VIII of the Periodic Table and a carrier. Preferably, the isomerization catalyst is a catalyst containing SAPO-11 or SAPO-41 or ZSM-22 or ZSM-23 or ferrierite and Pt, Pd, or Ni and Al2O3 or SiO2. Typical Pt/SAPO-11/Al2O3, Pt/ZSM-22/Al2O3, Pt/ZSM-23/Al2O3 and/or Pt/SAPO-11/SiO2 are used as catalyst in the isomerization step (isomerization treatment). In certain embodiments, the hydrotreatment catalyst(s) and the isomerization catalyst(s) are not in contact with the reaction feed (the renewable feedstock and/or n-paraffins and/or i-paraffins derived therefrom) at the same time.

In certain embodiments, the renewable feedstock (renewable source(s) from which the renewable paraffinic diesel component is derived) comprises vegetable oil, or wood oil, or other plant based oil, or animal oil, or animal fat, or fish fat, or fish oil, or algae oil, or microbial oil, or a combination thereof. Optionally or additionally, the renewable feedstock may also comprise recyclable waste and/or recyclable residue. Recyclable waste comprises material such as used cooking oil, free fatty acids, palm oil byproducts or process side streams, sludge, and side streams from vegetable oil processing. Preferably, the renewable feedstock comprises at least one of vegetable oil, vegetable fat, animal oil, and animal fat. These materials are preferred, since they allow providing a renewable feedstock having a predictable composition which can be adjusted as needed by appropriate selection and optional blending of the natural oil(s) and/or fat(s). Further, renewable feedstock comprising recyclable waste, or recyclable residues, or both, is preferred, since recyclable waste and/or recyclable residues improve the overall sustainability of the renewable feedstock and consequently also of the paraffinic diesel component. Optionally, recyclable waste and/or recyclable residues may be combined with fresh feed of renewable oils and/or renewable fats, such as vegetable oil, vegetable fat, animal oil, and/or animal fat. Fresh feed refers herein to components that have not been recycled.

Glycerol tert-butyl ethers (GTBEs) can be formed by reacting glycerol (glycerin) with isobutene (isobutylene, i-butene). Neat glycerol is not suitable for fuel blending. Glycerol, having three hydroxyl (OH-) groups, may form mono-, di-, or tri- ethers with i-butene (mono-, di-, or tri-GTBE, respectively). The starting materials and products of said etherification reactions are schematically presented below.

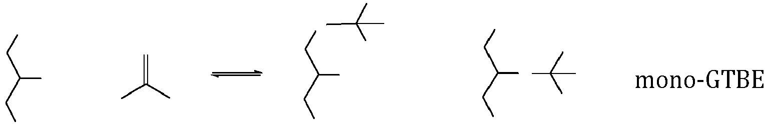

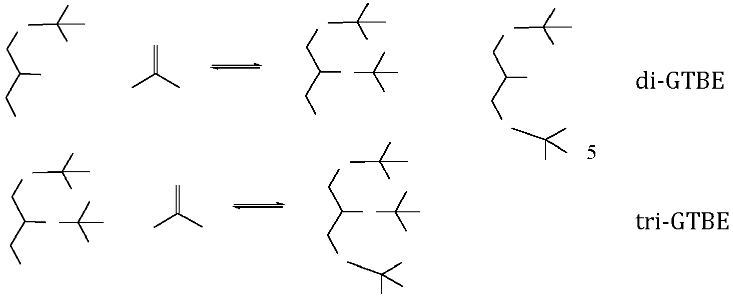

Glycerol may be derived from renewable sources, typically renewable oils and/or fats, such as animal fats and/or vegetable oils. Typically, glycerol is obtained from triglycerides through hydrolysis, saponification, or transesterification. Glycerol may also be formed as a by-product in industrial processes, such as soap manufacturing. Further, glycerol may sometimes be produced by microbial synthesis. A growing source of glycerol is biodiesel manufacturing, where glycerol is formed as a by-product. Biodiesel, or fatty acid methyl esters (FAME), are derived from renewable sources, typically though transesterification of renewable oils and/or fats. Thus, glycerol from a waste stream or side stream of the FAME production process is renewable (derived from renewable raw material). Converting glycerol from the FAME production process to di-GTBE gives said glycerol a value added use.

Isobutene used in the synthesis of di-GTBE can be derived from fossil and/or renewable sources. Petrochemical production of i-butene include cracking of petroleum products, preferably high-molecular weight hydrocarbon fractions derived from crude oil or other fossil sources. Isobutene may also be derived from tertiary butyl alcohol (TBA), methyl tert-butyl ether (MTBE), ethyl tert-butyl ether (ETBE), butane, isobutane, and/or isobutanol, which may originate from fossil and/or renewable sources. However, preferably, the i-butene used in the synthesis of di-GTBE is derived from renewable sources, for example by cracking hydrocarbon fractions derived from renewable raw material. Yet further, i-butene may be obtained through fermentation of sugar, typically glucose, sucrose, or hydrolysate of wood chip, straw, and/or bagasse.

In certain embodiments, providing di-GTBE comprises providing glycerol from a waste stream or side stream of fatty acid methyl esters (FAME) production, and subjecting said glycerol to an etherification treatment with isobutene derived from renewable sources to produce di-GTBE. The di-GTBE of such embodiments is renewable di-GTBE. Mixing renewable di-GTBE with the renewable paraffinic diesel component produces a renewable diesel fuel composition. Renewable diesel fuel compositions are preferred since they are more environmentally sustainable than non-renewable or fossil diesel fuel compositions. The diesel fuel composition of the present invention may thus be a renewable diesel fuel composition obtainable or obtained by blending the renewable paraffinic diesel component with renewable di-GTBE. In certain embodiments, the diesel fuel composition of the present invention is a renewable diesel fuel composition without components derived from fossil sources.

In certain embodiments, providing glycerol from a waste stream or side stream of fatty acid methyl esters (FAME) production comprises purifying said glycerol before reacting it with i-butene to form di-GTBE. In certain embodiments, providing di-GTBE comprises performing the etherification treatment in the presence of an acid catalyst, such as p-toluene sulfonic acid, sulfonic acid, sulfosuccinic acid, or Amberlyst^{®} catalysts.

The present invention further provides use of di-glycerol tert-butyl ether (di-GTBE) as a lubricity improver for fuels. It has surprisingly been found that adding di-GTBE to a fuel yields a combined fuel composition with improved lubricity properties compared to the lubricity properties of the fuel before the addition of di-GTBE. In certain embodiments, di-GTBE is used as a lubricity improver for renewable diesel fuels, preferably renewable paraffinic diesel fuels.

The present invention also provides a method for decreasing High Frequency Reciprocating Rig (HFRR) -lubricity, measured according to EN ISO12156-1:2016, of a fuel, the method comprising adding di-GTBE to the fuel. It has surprisingly been found that the HFRR-lubricity of a fuel can be decreased by adding di-GTBE to it. In certain embodiments, the method is a method for decreasing the High Frequency Reciprocating Rig (HFRR) -lubricity, measured according to EN ISO12156-1:2016, of a renewable diesel fuel, the method comprising adding di-GTBE to the renewable diesel fuel. In certain embodiments, the method is a method for decreasing High Frequency Reciprocating Rig (HFRR) -lubricity, measured according to EN ISO12156-1:2016, of a renewable paraffinic diesel fuel, the method comprising adding di-GTBE to the renewable paraffinic diesel fuel.

In certain embodiments, di-GTBE is added to the fuel to form a combined fuel composition comprising, based on the total combined fuel composition volume, at least 5 vol-% di-GTBE and at most 95 vol-% of said fuel. It has surprisingly been found that adding at least 5 vol-% di-GTBE yields combined fuel compositions having a HRFF-lubricity according to EN ISO12156-1:2016 below 460 µm.

In certain embodiments, di-GTBE is added to the fuel to form a combined fuel composition comprising, based on the total combined fuel composition volume, at least 10 vol-% di-GTBE and at most 90 vol-% of said fuel. It has surprisingly been found that adding at least 10 vol-% di-GTBE yields combined fuel compositions having a HRFF-lubricity according to EN ISO12156-1:2016 below 400 µm.

In certain embodiments, di-GTBE is added to the fuel to form a combined fuel composition comprising, based on the total combined fuel composition volume, 5-50 vol-% di-GTBE and 50-95 vol-% of said fuel, or 5-40 vol-% di-GTBE and 60-95 vol-% of said fuel, preferably 5-30 vol-% di-GTBE and 70-95 vol-% of said fuel, further preferably 5-25 vol-% di-GTBE and 75-95 vol-% of said fuel, more preferably 5-20 vol-% di-GTBE and 80-95 vol-% of said fuel. Adding such amounts of di-GTBE to the fuel yields a combined fuel composition having a HRFF-lubricity below 460 µm and good compatibility with diesel fuel engines.

In certain embodiments, di-GTBE is added to the fuel to form a combined fuel composition comprising, based on the total combined fuel composition volume, 5-50 vol-% di-GTBE and 50-95 vol-% of said fuel, or 10-40 vol-% di-GTBE and 60-90 vol-% of said fuel, preferably 10-30 vol-% di-GTBE and 70-90 vol-% of said fuel, further preferably 10-25 vol-% di-GTBE and 75-90 vol-% of said fuel, more preferably 10-20 vol-% di-GTBE and 80-90 vol-% of said fuel. Adding such amounts of di-GTBE to the fuel yields a combined fuel composition having a HRFF-lubricity below 400 µm and good compatibility with diesel fuel engines.

In certain embodiments, di-GTBE is added to the fuel to form a combined fuel composition comprising, based on the total combined fuel composition volume, 5-22 vol-% di-GTBE and 78-95 vol-% of said fuel, or 5-18 vol-% di-GTBE and 82-95 vol-% of said fuel, preferably 5-15 vol-% di-GTBE and 85-95 vol-% of said fuel, further preferably 5-13 vol-% di-GTBE and 87-95 vol-% of said fuel, more preferably 7-11 vol-% di-GTBE and 89-93vol-% of said fuel, even more preferably 8-10 vol-% di-GTBE and 90-92 vol-% of said fuel. Adding di-GTBE to the fuel in such amounts is enough to yield a combined diesel fuel composition with beneficial lubricity properties.

In certain embodiments, di-GTBE is added to the fuel to form a combined fuel composition comprising, based on the total combined fuel composition volume, 10-22 vol-% di-GTBE and 78-90 vol-% of said fuel, or 10-19 vol-% di-GTBE and 81-90 vol-% of said fuel, preferably 10-17 vol-% di-GTBE and 83-90 vol-% of said fuel, further preferably 10-15 vol-% di-GTBE and 85-90 vol-% of said fuel, more preferably 10-12 vol-% di-GTBE and 88-90 vol-% of said fuel. Adding di-GTBE to the fuel in such amounts is enough to yield a combined diesel fuel composition with very beneficial lubricity properties.

In certain embodiments di-GTBE is added to the fuel to form a combined fuel composition comprising, based on the total combined fuel composition volume, 13-22 vol-% di-GTBE and 78-87 vol-% of said fuel.

Di-GTBE may be added to the fuel to form a combined fuel composition in which the combined amount of di-GTBE and the fuel is at least 95 vol-%, preferably at least 97 vol-%, more preferably at least 99 vol-% of the total combined fuel composition volume. Adding di-GTBE to the fuel to form combined diesel fuel compositions with a high combined amount of di-GTBE and the fuel results in combined fuel compositions that perform well as a diesel fuel and have beneficial lubricity properties. These effects are further favored as the combined amount of the renewable paraffinic diesel component and di-GTBE in the combined diesel fuel composition increases. In certain embodiments, the combined fuel composition based on which the vol-% are expressed consists essentially of di-GTBE and fuel components, such as fossil diesel, biodiesel, and/or renewable (hydrotreated) diesel. In certain embodiments, the combined fuel composition consists essentially of di-GTBE and the fuel (to which di-GTBE was added).

In certain embodiments, the fuel is renewable diesel fuel, preferably renewable paraffinic diesel fuel, i.e. in certain embodiments, the combined fuel composition consists essentially of di-GTBE and renewable diesel fuel, preferably renewable paraffinic diesel fuel. An example of a renewable paraffinic diesel fuel is the renewable paraffinic diesel component described herein.

### EXAMPLES

The following examples are provided to better illustrate the claimed invention and are not to be interpreted as limiting the scope of the invention. To the extent that specific materials are mentioned, it is merely for purposes of illustration and is not intended to limit the invention.

Mono-glycerol tert-butyl ether (mono-GTBE), di-glycerol tert-butyl ether (di-GTBE), tri-glycerol tert-butyl ether (tri-GTBE), 1-octanol, and di-n-pentyl ether (DNPE) where studied as potential components to be blended with a renewable paraffinic diesel component to obtain a diesel fuel composition with beneficial lubricity properties.

Mono-GTBE, di-GTBE, and tri-GTBE used in these examples were provided by Procede group BV. As paraffinic renewable diesel component three batches of Neste Renewable Diesel (referred also to as renewable paraffinic diesel or renewable diesel in the following) were used. Said Neste Renewable Diesel batches had different lubricity values.

It was found that mono-GTBE was not miscible with the renewable paraffinic diesel and accordingly, could not be blended with renewable paraffinic diesel to form a sound diesel fuel composition. Without being bound to any theory, it is believed that mono-GTBE, comprising two hydroxyl groups, is too polar to blend with renewable paraffinic diesel comprising mainly high-molecular weight paraffins. Hence, mono-GTBE, and blends of mono-GTBE and renewable paraffinic diesel, were not studied further. The other blending components, namely di-GTBE, tri-GTBE, 1-octanol, and DNPE, where found to be miscible with renewable paraffinic diesel, and the lubricity properties of such blends were studied.

The lubricity properties were studied with High Frequency Reciprocating Rig (HFRR) -measurements. Said measurements were performed according to standard EN ISO 12156-1:2016. The measured lubricity values are given in microns (µm).

The lubricities of neat di-GTBE and neat tri-GTBE were measured. The results are presented in table 1. Both di-GTBE and tri-GTBE were found to have beneficial lubricity values, 300 µm and 369 µm respectively. Both values are well below the maximum value 460 µm set by EN 590:2017 for automotive diesel fuel and EN 15940:2016 for automotive paraffinic diesel fuel from synthesis or hydrotreatment. Further, the lubricity of each batch of neat renewable paraffinic diesel was measured. Batch 1 had a lubricity value of 591 µm, batch 2 had a lubricity value of 612 µm, and batch 3 had a lubricity value of 613 µm. All three renewable paraffinic diesel batches thus had lubricity values above the maximum value of EN 590:2017 and EN 15940:2016, thereby not meeting the requirements set by these standards.

Blends of di-GTBE and tri-GTBE were formed with batch 1 of paraffinic renewable diesel. Blends of 1-octanol were formed with all three batches of paraffinic renewable diesel, and blends of DNPE were formed with batch 2 and batch 3 of renewable paraffinic diesel. Table 1 and Fig. 1 presents measured lubricity values for blends of renewable paraffinic diesel and a second component being either di-GTBE, tri-GTBE, 1-octanol, or DNPE. As seen in Table 1 and Fig. 1, the selected renewable diesel batch did not importantly affect the lubricity values of the blends.

Fig. 1 plots the lubricity values of the above blends as a function of the vol-% of the second component based on the total blend volume. In Fig. 1, the diamonds denote blends of batch 1 paraffinic diesel component and di-GTBE, the squares denote blends of batch 1 paraffinic diesel component and tri-GTBE, the triangles denote blends of batch 3 paraffinic diesel component and 1-octanol, the x-symbols denote blends of batch 2 paraffinic diesel component and 1-octanol, the oblongs (rectangles) denote blends of batch 1 paraffinic diesel component and 1-octanol, the circles denote blends of batch 3 paraffinic diesel component and DNPE, and the crosses (+-symbols) denote blends of batch 2 paraffinic diesel component and DNPE.

In table 1, the compositions of the blends are shown on the left in vol-%, based on the total volume of the blend, and on the right, the second component of each blend together with the measured lubricity value of said blend are shown.

**Table 1. Results of High Frequency Reciprocating Rig (HFRR) -measurements according to EN ISO 12156-1:2016. The lubricity values are given in µm.**

| Renewable paraffinic diesel component, vol-% | Second component, vol-% | Second Component and Lubricity of Blend | | | | | | |
|---|---|---|---|---|---|---|---|---|
| | | di-GTBE | tri-GTBE | 1-octanol | 1-octanol | 1-octanol | DNPE | DNPE |
| 100* | 0 | 591 | 591 | 613 | 612 | 591 | 613 | 612 |
| 90 | 10 | 356 | 628 | 537 | 545 | | 585 | 616 |
| 80 | 20 | 370 | 593 | 528 | 539 | 543 | 663 | 659 |
| 70 | 30 | 351 | 435 | | | | | |
| 0 | 100 | 300 | 369 | | | | | |

| | | | | | | | | |
|---|---|---|---|---|---|---|---|---|
| *the three batches of renewable paraffinic diesel (batch 1, batch 2, and batch 3) are recognized by their lubricity value. Batch 1 has a lubricity of 591 µm, batch 2 has a lubricity of 612 µm, and batch 3 has a lubricity of 613 µm at 100 vol-% renewable paraffinic diesel component. | | | | | | | | |

Without being bound to any theory, comparing the lubricity measurement results of Table 1 and Fig. 1 of blends comprising DNPE having one ether functionality (C-O-C), 1-octanol having one alcohol functionality (hydroxyl group, OH-), and tri-GTBE having three ether functionalities, it seems like ether functionality results in less beneficial lubricity properties, while alcohol functionality results in more beneficial lubricity properties. Looking at the lubricity values of blends comprising 10 vol-% or 20 vol-% of DNPE, 1-octanol, or tri-GTBE, blends of DNPE had the least favorable lubricity properties, while blends of 1-octanol had more beneficial lubricity properties than blends comprising DNPE or tri-GTBE. However, rather surprisingly, blends of di-GTBE, having two ether functionalities and one alcohol functionality, had the most beneficial lubricity properties.

At all vol-% of Table 1, the measured lubricity values of blends of di-GTBE and renewable paraffinic diesel had a lubricity value below 400 µm. Without being bound to any theory, it is believed that the beneficial lubricity properties of blends of renewable paraffinic diesel and di-GTBE are due to the combination of the ether and alcohol functionalities of di-GTBE, which are believed to offer surface active characteristics for lubricity purposes. Whereas neat di-GTBE and neat tri-GTBE both show good lubricity properties, surprisingly, as blends with renewable paraffinic diesel, particularly at 10 vol-% and 20 vol-%, blends of di-GTBE and renewable diesel had significantly better lubricity values than the blends of tri-GTBE and renewable diesel.

Fig. 2 presents measured lubricity values of blends of di-GTBE and renewable paraffinic diesel as a function of vol-% of di-GTBE in the blend based on the total volume of the blend. Surprisingly, the decrease in the measured lubricity was not a linear function of the vol-% of di-GTBE in the blend. In fact, compared with the significant drop from 591 µm of neat renewable paraffinic diesel component to 356 µm of a blend comprising 10 vol-% di-GTBE and 90 vol-% renewable paraffinic diesel, increasing the vol-% of di-GTBE in the blend to vol-% above 10 improved the lubricity of the blend just slightly. Blends comprising approximately 10 vol-% di-GTBE and 90 vol-% renewable paraffinic diesel are thus of particular interest.

The density of neat di-GTBE at 15 °C was approximately 904 kg/m³ which is above the maximum density values of current fuel standards EN 590:2017 (maximum allowed density 845 kg/m³) and EN 15940:2016 category B (maximum allowed density 810 kg/m³). The density of neat paraffinic renewable diesel at 15 °C was approximately 780 kg/m³, which is below the minimum density allowed by EN 590:2017, namely 800 kg/m³, but fulfills the density requirements of EN 15940:2016 category B, namely 780-810 kg/m³. Fig. 3 shows calculated density values of blends of di-GTBE and renewable paraffinic diesel. The density values are plotted in Fig. 3 as a function of the wt-% of di-GTBE in the blend based on the total weight of the blend. In Fig. 3, the horizontal axis is labeled such that 1 corresponds to 100 wt-%, 0.7 to 70 wt-%, 0.5 to 50 wt-% and so on.

As seen in Fig. 3, blends comprising 15-55 wt-% di-GTBE (corresponding to 13-51 vol-% di-GTBE) and 45-85 wt-% renewable paraffinic diesel (corresponding to 49-87 vol-% renewable paraffinic diesel) fulfill the density requirements of standard EN 590:2017, namely 800-845 kg/m³. Further, as seen in Fig. 3, blends comprising 0-25 wt-% di-GTBE (corresponding to 0-22 vol-% di-GTBE) and 75-100 wt-% renewable paraffinic diesel (corresponding to 78-100 vol-% renewable paraffinic diesel) fulfill the density requirements of standard EN 15940:2016 category B, namely 780-810 kg/m³. Consequently, blends comprising 15-25 wt-% di-GTBE (corresponding to 13-22 vol-% di-GTBE) and 75-85 wt-% renewable paraffinic diesel (corresponding to 78-87 vol-% renewable paraffinic diesel component) fulfill the density requirements of both EN 590:2017 and EN 15940:2016 category B.

Thus, blends comprising 5-22 vol-% di-GTBE and 78-95 vol-% renewable paraffinic diesel had good lubricity properties without exceeding the maximum allowed density of EN 15940:2016 category B. However, by increasing the vol-% of di-GTBE and decreasing the amount of renewable paraffinic diesel in the blend to 13-51 vol-% di-GTBE and 49-87 vol-% renewable paraffinic diesel, the good lubricity properties were maintained or improved and the density requirements of EN 590:2017 were fulfilled. Rather surprisingly, blends comprising 13-22 vol-% di-GTBE and 78-87 vol-% renewable paraffinic diesel had particularly good lubricity properties while fulfilling the density requirements of both EN 15940:2016 category B and EN 590:2017.

The foregoing description has provided by way of non-limiting examples of particular implementations and embodiments of the invention a full and informative description of the best mode presently contemplated by the inventors for carrying out the invention. The scope of the invention is only restricted by the appended patent claims.

## Claims

1. A diesel fuel composition comprising:
a. a renewable paraffinic diesel component at most 95 vol-% of the total diesel fuel composition volume, the renewable paraffinic diesel component comprising paraffins in the range of carbon number C15-C18 at least 70 wt-% of the total weight of paraffins in the renewable paraffinic diesel component; and
b. di-glycerol tert-butyl ether (di-GTBE) at least 5 vol-% of the total diesel fuel composition volume.

2. The diesel fuel composition according to claim 1, comprising di-GTBE 5-50 vol-%, preferably 5-40 vol-%, further preferably 5-30 vol-%, yet further preferably 5-25 vol-%, more preferably 5-20 vol-%, and even more preferably 5-15 vol-% of the total diesel fuel composition volume.

3. The diesel fuel composition according to any of the preceding claims, comprising di-GTBE 10-50 vol-%, preferably 10-40 vol-%, further preferably 10-30 vol-%, yet further preferably 10-25 vol-%, more preferably 10-20 vol-%, and even more preferably 10-15 vol-% of the total diesel fuel composition volume.

4. The diesel fuel composition according to any of the preceding claims, comprising renewable paraffinic diesel component 50-95 vol-% or 50-90 vol-%, further preferably 60-95 vol-% or 60-90 vol-%, yet further preferably 70-95 vol-% or 70-90 vol-%, more preferably 75-95 vol-% or 75-90 vol-%, even more preferably 80-95 vol-% or 80-90 vol-%, and most preferably 85-95 vol-% or 85-90 vol-% of the total diesel fuel composition volume.

5. The diesel fuel composition according to any of the preceding claims, wherein the combined amount of a. and b. in the diesel fuel composition is at least 95 vol-%, more preferably at least 97 vol-%, even more preferably at least 99 vol-% of the total diesel fuel composition volume.

6. The diesel fuel composition according to any of the preceding claims, wherein the renewable paraffinic diesel component comprises paraffins at least 90 wt-%, preferably at least 95 wt-%, and more preferably at least 99 wt-% of the total weight of the renewable paraffinic diesel component.

7. The diesel fuel composition according to any of the preceding claims, wherein the renewable paraffinic diesel component comprises paraffins in the range of carbon number C15-C18 at least 80 wt-%, preferably at least 90 wt-% of the total weight of paraffins in the renewable paraffinic diesel component.

8. The diesel fuel composition according to any of the preceding claims, wherein the renewable paraffinic diesel component comprises i-paraffins and n-paraffins in a weight ratio of i-paraffins to n-paraffins of at least 2.2, at least 2,3, at least 3 or at least 4.

9. A method for producing a diesel fuel composition, comprising:
providing a renewable paraffinic diesel component comprising;
i. providing a feedstock originating from renewable sources, the feedstock comprising fatty acids, or triglycerides, or both;
ii. subjecting the renewable feedstock to hydrotreatment, preferably hydro deoxygenation, to produce n-paraffins; and optionally
iii. subjecting at least a portion of the n-paraffins from step ii) to an isomerization treatment to produce i-paraffins;
providing di-glycerol tert-butyl ether (di-GTBE); and
mixing the renewable paraffinic diesel component with the di-GTBE to form a diesel fuel composition comprising di-GTBE at least 5 vol-% and renewable paraffinic diesel component at most 95 vol-% of the total diesel fuel composition volume.

10. The method according to claim 9, wherein providing di-GTBE comprises:
I. providing glycerol from a waste stream or a side stream of fatty acid methyl esters (FAME) production; and
II. subjecting said glycerol to an etherification treatment with iso-butene derived from renewable sources to produce di-GTBE.

11. Use of a diesel fuel composition according to any of claims 1 to 8 as a fuel in a diesel engine.

12. Use of di-glycerol tert-butyl ether (di-GTBE) as a lubricity improver for fuels.

13. The use of claim 12, wherein di-GTBE is used as a lubricity improver for renewable diesel fuels, preferably renewable paraffinic diesel fuels.

14. The use of claim 12 or 13, wherein di-GTBE is added to the fuel to form a combined fuel composition comprising at least 5 vol-%, preferably at least 10 vol-%, di-GTBE of the total combined fuel composition volume.

## Patentansprüche

1. Dieselkraftstoffzusammensetzung, umfassend:
a. eine erneuerbare paraffinische Dieselkomponente, die höchstens 95 Vol.-% des Gesamtvolumens der Dieselkraftstoffzusammensetzung ausmacht, wobei die erneuerbare paraffinische Dieselkomponente Paraffine im Bereich der Kohlenstoffzahl C15-C18 umfasst, die mindestens 70 Gew.-% des Gesamtgewichts der Paraffine in der erneuerbaren paraffinischen Dieselkomponente ausmachen; und
b. Di-Glycerin-tert.-butylether (di-GTBE) mindestens 5 Vol.-% des Gesamtvolumens der Dieselkraftstoffzusammensetzung.

2. Dieselkraftstoffzusammensetzung nach Anspruch 1, die di-GTBE zu 5-50 Vol.-%, bevorzugt 5-40 Vol.-%, weiter bevorzugt 5-30 Vol.-%, noch weiter bevorzugt 5-25 Vol.-%, bevorzugter 5-20 Vol.-% und noch bevorzugter 5-15 Vol.-% des Gesamtvolumens der Dieselkraftstoffzusammensetzung umfasst.

3. Dieselkraftstoffzusammensetzung nach einem der vorangehenden Ansprüche, die di-GTBE zu 10-50 Vol.-%, bevorzugt 10-40 Vol.-%, weiter bevorzugt 10-30 Vol.-%, noch weiter bevorzugt 10-25 Vol.-%, bevorzugter 10-20 Vol.-% und noch bevorzugter 10-15 Vol.-% des Gesamtvolumens der Dieselkraftstoffzusammensetzung umfasst.

4. Dieselkraftstoffzusammensetzung nach einem der vorangehenden Ansprüche, die eine erneuerbare paraffinische Dieselkomponente zu 50-95 Vol.-% oder 50-90 Vol.-%, weiter bevorzugt 60-95 Vol.-% oder 60-90 Vol.-%, noch weiter bevorzugt 70-95 Vol.-% oder 70-90 Vol.-%, bevorzugter 75-95 Vol.-% oder 75-90 Vol.-%, noch bevorzugter 80- 95 Vol.-% oder 80-90 Vol.-% und am meisten bevorzugt 85-95 Vol.-% oder 85-90 Vol.-% des Gesamtvolumens der Dieselkraftstoffzusammensetzung umfasst.

5. Dieselkraftstoffzusammensetzung nach einem der vorhergehenden Ansprüche, wobei die kombinierte Menge von a. und b. in der Dieselkraftstoffzusammensetzung mindestens 95 Vol.-%, bevorzugter mindestens 97 Vol.-% und noch bevorzugter mindestens 99 Vol.-% des Gesamtvolumens der Dieselkraftstoffzusammensetzung beträgt.

6. Dieselkraftstoffzusammensetzung nach einem der vorhergehenden Ansprüche, wobei die erneuerbare paraffinische Dieselkomponente mindestens 90 Gew.-%, bevorzugt mindestens 95 Gew.-% und besonders bevorzugt mindestens 99 Gew.-% des Gesamtgewichts der erneuerbaren paraffinischen Dieselkomponente Paraffine umfasst.

7. Dieselkraftstoffzusammensetzung nach einem der vorhergehenden Ansprüche, wobei die erneuerbare paraffinische Dieselkomponente Paraffine im Bereich der Kohlenstoffzahl C15-C18 zu mindestens 80 Gew.-%, bevorzugt zu mindestens 90 Gew.-% des Gesamtgewichts der Paraffine in der erneuerbaren paraffinischen Dieselkomponente umfasst.

8. Dieselkraftstoffzusammensetzung nach einem der vorhergehenden Ansprüche, wobei die erneuerbare paraffinische Dieselkomponente i-Paraffine und n-Paraffine in einem Gewichtsverhältnis von i-Paraffinen zu n-Paraffinen von mindestens 2,2, mindestens 2,3, mindestens 3 oder mindestens 4 umfasst.

9. Verfahren zur Herstellung einer Dieselkraftstoffzusammensetzung, umfassend:
Bereitstellen einer erneuerbaren paraffinischen Dieselkomponente, umfassend;
i. Bereitstellen eines aus erneuerbaren Quellen stammenden Ausgangsmaterials, wobei das Ausgangsmaterial Fettsäuren oder Triglyceride oder beides umfasst;
ii. Unterziehen des erneuerbaren Ausgangsmaterials einer Hydrobehandlung, bevorzugt Hydrodesoxydieren, um n-Paraffine zu erzeugen; und optional
iii. Unterziehen mindestens eines Teils der n-Paraffine aus Schritt ii) einer Isomerisierungsbehandlung zur Herstellung von i-Paraffinen;
Bereitstellen von Di-Glycerin-tert.-butylether (Di-GTBE); und
Mischen der erneuerbaren paraffinischen Dieselkomponente mit dem di-GTBE, um eine Dieselkraftstoffzusammensetzung zu bilden, die di-GTBE in einer Menge von mindestens 5 Vol.-% und eine erneuerbare paraffinische Dieselkomponente in einer Menge von höchstens 95 Vol.-% umfasst.

10. Verfahren nach Anspruch 9, wobei die Bereitstellung von di-GTBE umfasst:
I. Bereitstellen von Glycerin aus einem Abfallstrom oder einem Nebenstrom der Fettsäuremethylester (FAME)-Produktion; und
II. Unterziehen des Glycerins einer Veretherungsbehandlung mit IsoButen, das aus erneuerbaren Quellen stammt, um di-GTBE herzustellen.

11. Verwendung einer Dieselkraftstoffzusammensetzung nach einem der Ansprüche 1 bis 8 als Kraftstoff in einem Dieselmotor.

12. Verwendung von Di-Glycerin-tert-butylether (di-GTBE) als Schmierfähigkeitsverbesserer für Kraftstoffe.

13. Verwendung nach Anspruch 12, wobei di-GTBE als Schmierfähigkeitsverbesserer für erneuerbare Dieselkraftstoffe, bevorzugt erneuerbare paraffinische Dieselkraftstoffe, verwendet wird.

14. Verwendung nach Anspruch 12 oder 13, wobei di-GTBE dem Kraftstoff zugesetzt wird, um eine kombinierte Kraftstoffzusammensetzung zu bilden, die mindestens 5 Vol.-%, bevorzugt mindestens 10 Vol.-% des Gesamtvolumens der kombinierten Kraftstoffzusammensetzung di-GTBE umfasst.

## Revendications

1. Composition de carburant diesel comprenant :
a. un composé diesel paraffinique renouvelable représentant au plus 95 % en volume du volume total de la composition de carburant diesel, le composé dièse! paraffinique renouvelable comprenant des paraffines ayant un nombre de carbone dans la plage allant de C15 à C18 représentant au moins 70% en poids du poids total des paraffines dans le composé dièse! paraffinique renouvelable ; et
b. de l'éther de tert-butyle di-glycérol (di-GTBE) représentant au moins 5 % en volume du volume total de la composition de carburant diesel.

2. Composition de carburant diesel selon la revendication 1, comprenant du di-GTBE de 5 à 50 % en volume, de préférence de 5 à 40 % en volume, plus préférablement de 5 à 30 % en volume, encore plus préférablement de 5 à 25 % en volume, plus préférablement de 5 à 20 % en volume, et encore plus préférablement de 5 à 15 % en volume du volume total de la composition de carburant diesel.

3. Composition de carburant diesel selon l'une quelconque des revendications précédentes, comprenant du di-GTBE de 10 à 50 % en volume, de préférence de 10 à 40 % en volume, plus préférablement de 10 à 30 % en volume, encore plus préférablement de 10 à 25 % en volume, plus préférablement de 10 à 20 % en volume, et encore plus préférablement de 10 à 15 % en volume du volume total de la composition de carburant diesel.

4. Composition de carburant diesel selon l'une quelconque des revendications précédentes, comprenant un composé diesel paraffinique renouvelable de 50 à 95 % en volume ou de 50 à 90 % en volume, plus préférablement de 60 à 95 % en volume ou de 60 à 90 % en volume, encore plus préférablement de 70 à 95 % en volume ou de 70 à 90 % en volume, encore plus préférablement de 75 à 95 % en volume ou de 75 à 90 % en volume, encore plus préférablement de 80 à 95 % en volume ou de 80 à 90 % en volume, et le plus préférablement de 85 à 95 % en volume ou de 85 à 90 vol % en volume du volume total de la composition de carburant diesel.

5. Composition de carburant diesel selon l'une quelconque des revendications précédentes, dans laquelle la quantité combinée de a et b dans la composition de carburant diesel est d'au moins 95 % en volume, plus préférablement d'au moins 97 % en volume, encore plus préférablement d'au moins 99 % en volume du volume total de la composition de carburant diesel.

6. Composition de carburant diesel selon l'une quelconque des revendications précédentes, dans laquelle le composé diesel paraffinique renouvelable comprend des paraffines à au moins 90 % en poids, de préférence au moins 95 % en poids, et plus préférablement d'au moins 99 % en poids du poids total du composé diesel paraffinique renouvelable.

7. Composition de carburant diesel selon l'une quelconque des revendications précédentes, dans laquelle le composant diesel paraffinique renouvelable comprend des paraffines dans la plage de nombre de carbone dans la plage allant de C15 à C18 représentant au moins 70 % en poids, plus préférablement au moins 80 % en poids, le plus préférablement au moins 90 % en poids du poids total de paraffines dans le composant diesel paraffinique renouvelable.

8. Composition de carburant diesel selon l'une quelconque des revendications précédentes, dans laquelle le composé diesel paraffinique renouvelable comprend des i-paraffines et des n-paraffines dans un rapport pondéral des i-paraffines aux n-paraffines d'au moins 2,2, au moins 2,3, au moins 3 ou au moins 4.

9. Procédé de production d'une composition de carburant diesel, comprenant les étapes de
fournir un composé diesel paraffinique renouvelable comprenant les étapes de :
i. fournir une charge issue de sources renouvelables, la charge comprenant des acides gras, ou des triglycérides, ou les deux ;
ii. soumettre la charge d'alimentation renouvelable à un hydrotraitement, de préférence une hydrodésoxygénation, pour produire des n-paraffines ; et éventuellement
iii. soumettre au moins une partie des n-paraffines de l'étape ii. à un traitement d'isomérisation pour produire des i-paraffines ;
fournir de l'éther de tert-butyle di-glycérol (di-GTBE); et
mélanger le composé diesel paraffinique renouvelable avec le di-GTBE pour former une composition de carburant diesel comprenant du di-GTBE à au moins 5 % en volume et un composant diesel paraffinique renouvelable à au plus 95 % en volume du volume total de la composition de carburant diesel.

10. Procédé selon la revendication 9, dans lequel l'étape de fournir du di-GTBE comprend les étapes de:
I. fournir du glycérol à partir d'un flux de déchets ou d'un flux latéral de production d'esters méthyliques d'acides gras (FAME) ; et
II. soumettre ledit glycérol à un traitement d'éthérification avec de l'iso-butène dérivé de sources renouvelables pour produire du di-GTBE.

11. Utilisation d'une composition de carburant diesel selon l'une quelconque des revendications 1 à 8 comme carburant dans un moteur diesel.

12. Utilisation d'éther tert-butylique di-glycérol (di-GTBE) comme agent améliorant le pouvoir lubrifiant pour carburants.

13. Utilisation selon la revendication 12, dans laquelle le di-GTBE est utilisé comme agent améliorant le pouvoir lubrifiant pour des carburants diesel renouvelables, de préférence des carburants diesel paraffiniques renouvelables.

14. Utilisation selon la revendication 12 ou 13, dans laquelle du di-GTBE est ajouté au carburant pour former une composition de carburant combinée comprenant au moins 5 % en volume, de préférence au moins 10 % en volume, de di-GTBE du volume total de la composition de carburant combinée.
